# EUROPEAN PATENT SPECIFICATION

(11) **EP 2 844 391 B1**
(45) Date of publication and mention of the grant of the patent: **08.07.2020**
(21) Application number: 13721335.1
(22) Date of filing: 03.05.2013
(51) Int. Cl.: B01J 37/02, B01J 37/16, B01J 37/18, B01J 23/44, B01J 23/52, B01J 23/58, B01J 35/00, C07C 67/055

(54) **METHOD FOR PRODUCING A SHELL CATALYST, THE CATALYST AND ITS USE FOR THE SYNTHESIS OF ALKENYL CARBOXYLIC ESTERS**
VERFAHREN ZUR HERSTELLUNG EINES SCHALENKATALYSATORS, BESAGTER KATALYSATOR UND DESSEN VERWENDUNG ZUR SYNTHESE VON ALKENYLCARBONSÄUREESTERN
PROCÉDÉ POUR LA PRODUCTION D'UN CATALYSEUR EN COQUILLE, CATALYSEUR ET SON UTILISATION POUR LA SYNTHÈSE D'ESTERS D'ACIDES ALCÉNYLCARBOXYLIQUES

(30) Priority: 03.05.2012 DE 102012008714
(43) Date of publication of application: 11.03.2015
(73) Proprietor: Clariant International Ltd, 4132 Muttenz (CH)
(72) Inventor: MESTL, Gerhard, 80935 München (DE); SCHECK, Peter, 82205 Gilching (DE); HAGEMEYER, Alfred, Sunnyvale, California 94086 (US); FISCHER, Carolin, 83024 Rosenheim (DE)
(74) Representative: Kuba, Stefan
(86) International application number: PCT/EP2013/059271
(87) International publication number: WO 2013/164458

(56) References cited:
- US-A- 5 347 046
- US-A- 5 693 586
- US-A1- 2005 181 940
- US-A1- 2010 273 644

## Description

The present invention relates to a method for producing a shell catalyst which is suitable for the synthesis of alkenyl carboxylic acid esters, in particular for producing vinyl acetate monomers (VAM) from ethylene and allyl acetate monomers from propylene by means of oxy-acetylation. The present invention also relates to a shell catalyst that can be obtained by the method according to the invention as well as the use of the shell catalyst produced using the method according to the invention or of the shell catalyst according to the invention for producing alkenyl carboxylic acid esters, in particular VAM and allyl acetate monomer.

Supported catalysts which contain palladium and gold have already been known for some time. VAM is usually produced in the presence of catalysts containing palladium and gold from a reaction mixture of ethylene, oxygen and acetic acid. Various production methods for such supported catalysts are already known. Thus, for example, precursor compounds which contain the corresponding metals are applied, dissolved preferably in an aqueous solution, to the surface of a support body. The support body containing the corresponding precursor compounds is then usually calcined under oxidizing conditions in a high-temperature oven, wherein the metal-containing precursor compounds are converted to the metal oxides. The support bodies which contain the corresponding metal oxides are then subjected to reduction to the elemental metals. In some known methods, however, precursor compounds are used in which an oxidation to the metal oxides is not necessary and the reduction step can be carried out directly after the drying. Vinyl acetate monomer is an important component for the production of polyvinyl acetate, vinyl acetate copolymers (such as ethylene vinyl acetates or ethylene vinyl alcohol copolymers) and polyvinyl alcohol. Because of the wide field of use of these polymers, for example as binders in the construction, paints, and varnishes sectors and as raw material for the adhesive, paper and textile industries, there is still a high demand for VAM and for constant improvement of the activity and selectivity of catalysts for their production.

Normally, in the synthesis of VAM, shell catalysts are used in which elemental palladium and gold are situated in an outer shell of the catalyst support body (hereafter called support body or shaped body). To produce them, a mixed solution of a Pd-containing precursor compound and an Au-containing precursor compound is normally applied to a catalyst support body which is then dried, and the metal components of the precursor compounds are converted to the elemental metals. The Pd/Au combination normally leads to a good selectivity or activity of the catalyst. In addition, due to the capital intensity of VAM production plants and increasingly high raw material costs, in particular for ethylene, there is a constant requirement to optimize the economic efficiency of the method for producing VAM by means of improved catalysts.

The object of the present invention was therefore to provide a method for producing a shell catalyst which results in a shell catalyst that outperforms previous catalysts in respect of the activity and selectivity in the synthesis of alkenyl carboxylic acid esters.

Furthermore, all the conventional methods for producing vinyl acetate and allyl acetate catalysts have proved to be capable of improvement in respect of their noble metal yield. The ratio between the proportion of noble metal, thus Pd and Au, which ultimately remains on the catalyst after all the production steps and the proportion of noble metal used is considered to be the noble metal yield. It was therefore also an object of the present invention to improve the noble metal yield in the production of corresponding catalysts.

These objects were achieved by a method according to the invention with which shell catalysts with significantly increased selectivity and activity for VAM can be produced. The method according to the invention for producing a shell catalyst is characterized by the following method steps:
(a) applying a solution containing an acetate to a support body, wherein the acetate contains an acetate anion, and afterwards drying the support body at a temperature in the range of from 70 to 120 °C;
(b) applying a Pd precursor compound and an Au precursor compound to a support body obtained after step (a) and impregnated with the acetate ;
(c) reducing the metal components of the precursor compounds of the support body obtained after step (b) to the elemental metals.

By the term "shell catalyst" is meant a catalyst which comprises a support body and a shell with catalytically active material, wherein the shell can be formed in two different ways:
Firstly, a catalytically active material can be present in the outer area of the support body, with the result that the material of the support body serves as matrix for the catalytically active material and the area of the support body which is impregnated with the catalytically active material forms a shell around the unimpregnated core of the support body. Secondly, an additional layer in which a catalytically active material is present can be applied to the surface of the support body. This layer thus forms an additional material layer which is constructed as a shell around the support body. In the latter variant, the support body material is not a constituent of the shell, but the shell is formed by the catalytically active material itself or a matrix material which comprises a catalytically active material. In the present invention, the first-named variant of a shell catalyst is preferred.

In the shell catalyst produced by the method according to the invention, the metals are present either in monoatomic form or in the form of aggregates. However, they are preferably present in the form of aggregates. The monoatomic atoms or multiatomic aggregates are dispersed predominantly uniformly inside the shell of the shell catalyst. By a multiatomic aggregate is meant the clustering of several metal atoms to form a composite which lies between monoatomic form and metallic type (alloy). The term also includes so-called metal clusters.

The shell thickness of the outer shell of the support body is preferably 1 to 50%, more preferably 2 to 40%, even more preferably 3 to 30% and most preferably 4 to 20% of half of the total thickness of the support body. The named percentage therefore relates to half of the total thickness as, depending on the shape of the support body during production, e.g. by spray impregnation with a solution containing precursor compound, the precursor compound either penetrates the support body material from two outer surfaces (sphere) or, if the support body material has a more complex shape, such as e.g. that of a hollow cylinder, there are an outer surface and an inner surface which the precursor compound penetrates. In the case of support body materials deviating from sphere geometry the total thickness of the support is measured along the longest support body axis. The outer shell boundary is equalized with the outer boundary of the metal-containing support body. By inner shell boundary is meant the boundary, located inside the support body, of the metal-containing shell which is at such a distance from the outer shell boundary that 95 wt.-% of all of the metal contained in the support body is located in the outer shell. However, the shell thickness is preferably not more than 50%, more preferably not more than 40%, even more preferably not more than 30% and most preferably not more than 20%, in each case relative to half of the total thickness of the support body.

The metal-impregnated support body preferably contains no more than 5% of the total metal in its inner area, thus inside the area that is delimited to the outside by the inner shell boundary of the metal shell.

With regard to the shell thickness of the catalyst, the maximum concentration of metal preferably lies in the area of the outer shell, particularly preferably at the outer edge of the outer shell, i.e. close to the geometric catalyst surface. The metal concentration preferably decreases towards the inner shell boundary.

The support body preferably consists of an inert material. It can be porous or non-porous. However, the support body is preferably porous. The support body preferably consists of particles with a regular or irregular shape, such as for example spheres, tablets, cylinders, solid cylinders or hollow cylinders, rings, stars or other shapes, and its dimensions, such as e.g. diameter, length or width, are in a range of from 1 to 10 mm, preferably 3 to 9 mm. Spherical, i.e. e.g. sphere-shaped, particles with a diameter of from 3 to 8 mm are preferred according to the invention. The support body material can be composed of any non-porous and porous substance, preferably porous substance. Examples of materials for this are titanium oxide, silicon oxide, aluminium oxide, zirconium oxide, magnesium oxide, silicon carbide, magnesium silicate, zinc oxide, zeolites, sheet silicates and nanomaterials, such as for example carbon nanotubes or carbon nanofibres.

The above-named oxidic support body materials can be used for example in the form of mixed oxides or defined compositions, such as for example TiO₂, SiO₂, Al₂O₃, ZrO₂, MgO, SiC or ZnO. Furthermore, soots, ethylene black, charcoal, graphite, hydrotalcites or further support body materials known per se to a person skilled in the art can preferably be used in different possible modifications. The support body materials can preferably be doped for instance with alkali or alkaline earth metals or also with phosphorus, halide and/or sulphate salts. The support body preferably comprises an Si-Al mixed oxide, or the support body consists of an Si-Al mixed oxide. The support body, preferably an Si-Al mixed oxide, can in addition also be doped with Zr and preferably contains this in a proportion of from 5 to 30 wt.-%, relative to the total weight of the support body.

The BET surface area of the support body material without the coating with the precursor compounds is 1 to 1,000 m²/g, preferably 10 to 600 m²/g, particularly preferably 20 to 400 m²/g and quite particularly preferably between 80 and 170 m²/g. The BET surface area is determined using the 1-point method by adsorption of nitrogen in accordance with DIN 66132.

In addition, it can be preferred that the integral pore volume of the support body material (determined in accordance with DIN 66133 (Hg porosimetry)) without the coating with the precursor compound is greater than 0.1 ml/g, preferably greater than 0.18 ml/g.

The support body is usually produced by subjecting a plurality of support bodies to a "batch" process, in the individual method steps of which the shaped bodies are subject to relatively high mechanical stresses for example by using stirring and mixing tools. In addition, the shell catalyst produced by the method according to the invention can be subjected to a strong mechanical load stress during the filling of a reactor, which can result in an undesired formation of dust as well as damage to the support body, in particular to its catalytically active shell located in an outer area.

In particular, to keep the abrasion of the catalyst produced by the method according to the invention within reasonable limits, the shell catalyst has a hardness greater than/equal to 20 N, preferably greater than/equal to 25 N, further preferably greater than/equal to 35 N and most preferably greater than/equal to 40 N. The hardness is ascertained by means of an 8M tablet-hardness testing machine from Dr. Schleuniger Pharmatron AG, determining the average for 99 shell catalysts, after drying of the catalyst at 130°C for 2 hours, wherein the apparatus settings are as follows:
Distance from the shaped body: 5.00 mm
Time delay: 0.80 s
Feed type: 6 B
Speed: 0.60 mm/s

The hardness of the shell catalyst produced by the method according to the invention can be influenced for example by means of variations in certain parameters of the method for producing the support body, for example by the calcining time and/or the calcining temperature of the support body. The just-mentioned calcining is not a calcining of the support body impregnated with the metal-containing precursor compounds, but merely a calcining step for producing the support body before the precursor compounds are applied.

It is also preferred that 80% of the integral pore volume of the support body is formed by mesopores and macropores, preferably at least 85% and most preferably at least 90%. This counteracts a reduced activity, effected by diffusion limitation, of the catalyst produced by the method according to the invention, in particular in the case of metal-containing shells with relatively large thicknesses. By micropores, mesopores and macropores are meant in this case pores which have a diameter of less than 2 nm, a diameter of from 2 to 5 nm and a diameter of more than 50 nm respectively.

The activity of the shell catalysts produced by the method according to the invention normally depends on the quantity of the metal loading in the shell: As a rule, the more metal there is in the shell, the higher the activity. The thickness of the shell here has a smaller influence on the activity, but is a decisive variable with respect to the selectivity of the catalysts. With equal metal loading of the catalyst support, the smaller the thickness of the outer shell of the catalyst is, the higher the selectivity of the shell catalysts produced by the method according to the invention is in general. It is thus decisive to set an optimum ratio of metal loading to shell thickness in order to guarantee the highest possible selectivity with the highest possible activity. It is therefore preferred that the shell of the shell catalyst produced according to the invention has a thickness in the range of from 20 µm to 1,000 pm, more preferably from 30 µm to 800 pm, even more preferably from 50 µm to 500 µm and most preferably from 100 µm to 300 pm.

The thickness of the shell can be measured visually by means of a microscope. The area in which the metal is deposited appears black, while the areas free of noble metals appear white. As a rule, in the case of shell catalysts produced according to the invention the boundary between areas containing noble metals and areas free of them is very sharp and can be clearly recognized visually. If the above-named boundary is not sharply defined and accordingly not clearly recognizable visually, the thickness of the shell corresponds - as already mentioned - to the thickness of a shell, measured starting from the outer surface of the catalyst support, which contains 95% of the noble metal deposited on the support. In order to ensure a largely uniform activity of the catalyst produced by the method according to the invention over the thickness of the noble metal-containing shell, the noble metal concentration should vary only relatively little over the shell thickness. It is therefore preferred if, over an area of 90% of the shell thickness, wherein the area is at a distance of 5% of the shell thickness from each of the outer and inner shell limits, the profile of the noble metal concentration of the catalyst varies from the average noble metal concentration of this area by a maximum of +/- 20%, preferably by a maximum of +/- 15% and by preference by a maximum of +/- 10%. Such profiles can be achieved for example by means of physical deposition methods, such as spray impregnation of a solution containing the precursor compound onto support bodies circulated in a gas. The support bodies here are preferably located in a so-called fluidized bed or in a fluid bed, but all devices in which the support bodies can be swirled in a gas glide layer are also conceivable. The named shell profiles can particularly preferably be obtained by means of the spraying-on described further below in a fluidized bed, a fluid bed or an Innojet AirCoater. In the case of the shell catalysts produced according to the invention, the named distribution of the metal loading preferably describes a rectangular function, i.e. the concentration does not decrease or only decreases imperceptibly during the course into the inside of the support body and ends with a relatively "sharp" boundary (see above-named distribution parameters). In addition to the rectangular function, the metal loading inside the shell can however also describe a triangular or trapezium function in the case of which the metal concentration gradually decreases from the outside to the inside in the shell. However, a metal distribution according to the rectangular function is particularly preferred.

Contrary to the views held until now with respect to the production of VAM catalysts, the applicants of the present application have surprisingly discovered that the application of an acetate for example in the form of an alkali acetate to a support body before the application of the metal precursor compounds and before the reduction of the metal components of the precursor compounds leads to a VAM shell catalyst which has a much higher activity and selectivity than shell catalysts in which the acetate is applied after the application of the metal precursor compounds and/or after the reduction of the metal components of the precursor compounds.

The acetate to be used in step (a) of the method according to the invention is preferably an alkali or alkaline earth acetate, in particular an alkali acetate. The alkali acetate can be lithium acetate, sodium acetate, potassium acetate, caesium acetate or rubidium acetate, but preferably potassium acetate.

The Pd precursor compounds and Au precursor compounds used in the method according to the invention are preferably water-soluble compounds.

The Pd precursor compound used in the method according to the invention is preferably selected from: nitrate compounds, nitrite compounds, acetate compounds, tetraamine compounds, diamine compounds, hydrogen carbonate compounds and hydroxidic metallate compounds.

Examples of preferred Pd precursor compounds are water-soluble Pd salts. The Pd precursor compound is particularly preferably selected from the group consisting of Pd(NH₃)₄(HCO₃)₂, Pd(NH₃)₄(HPO₄), ammonium Pd oxalate, Pd oxalate, K₂Pd(oxalate)₂, Pd(II) trifluoroacetate, Pd(NH₃)₄(OH)₂, Pd(NO₃)₂, H₂Pd(OAc)₂(OH)₂, Pd(NH₃)₂(NO₂)₂, Pd(NH₃)₄(NO₃)₂, H₂Pd(NO₂)₄, Na₂Pd(NO₂)₄, Pd(OAc)₂ as well as freshly precipitated Pd(OH)₂.

If freshly precipitated Pd(OH)₂ is used, it is preferably produced as follows: A 0.1 to 40 wt.-% aqueous solution is preferably produced from tetrachloropalladate. A base, preferably an aqueous solution of potassium hydroxide, is then added to this solution until a brown solid, namely Pd(OH)₂ precipitates. To produce a solution for application to the catalyst support, the freshly precipitated Pd(OH)₂ is isolated, washed and dissolved in an aqueous alkaline solution. Dissolution preferably takes place at a temperature within the range of from 4 to 40°C, particularly preferably 15 to 25°C. A lower temperature is not possible because of the freezing point of water, a higher temperature brings with it the disadvantage that after a certain time Pd(OH)₂ precipitates again in the aqueous solution and does not dissolve.

A solution of the compound Pd(NH₃)₄(OH)₂ is preferably produced as follows: A precursor compound such as for example Na₂PdCl₄ is - as previously described - precipitated with potassium hydroxide solution to palladium hydroxide, preferably at pH 11 and room temperature, and the precipitate, after filtration and washing, is dissolved in aqueous ammonia (maximum 8.5%) to form Pd(NH₃)₄(OH)₂ (approx. 30 minutes at room temperature, approx. 2 hours at 60°C).

Furthermore the Pd-nitrite precursor compounds can also be used in the method according to the invention. Preferred Pd-nitrite precursor compounds are for example those which are obtained by dissolving Pd(OAc)₂ in an NaNO₂ or KNO₂ solution.

However, the above-named hydroxo complexes or hydroxo compounds are particularly preferably used as Pd precursor compounds. The compound Pd(NH₃)₄(OH)₂ is quite particularly preferably used as Pd precursor compound.

The Au precursor compounds used in the method according to the invention are preferably selected from: acetate compounds, nitrite or nitrate compounds and oxidic or hydroxidic metallate compounds.

Examples of preferred Au precursor compounds are water-soluble Au salts. The Au precursor compound is preferably selected from the group consisting of KAuO₂, NaAuO₂, LiAuO₂, RbAuO₂, CsAuO₂, Ba(AuO₂)₂, NaAu(OAc)₃(OH), KAu(NO₂)₄, KAu(OAc)₃(OH), LiAu(OAc)₃(OH), RbAu(OAc)₃(OH), CsAu(OAc)₃(OH), HAu(NO₃)₄ and Au(OAc)₃. It may be advisable to add the Au(OAc)₃ or one of the named aurates in each case freshly by precipitation as oxide or hydroxide from an auric acid solution, washing and isolating the precipitate as well as taking up same in acetic acid or alkali hydroxide respectively. One of the named alkali aurates is particularly preferably used as Au-containing precursor compound, which is used in dissolved form for application to the support. The production of a potassium aurate solution is known in the literature and can be carried out in accordance with the production methods disclosed in the documents WO99/62632 and US 6,015,769. The other alkali aurates can also be produced in the same way. It is particularly preferred that CsAuO₂ or its hydroxide dissolved in water (CsAu(OH)₄) is used as Au precursor compound in the method according to the invention. In particular, the use of CsAuO₂ means that the Au precursor compound does not penetrate further into the support body than the Pd precursor compound, which makes possible a uniform distribution of the two components in the shell.

The named precursor compounds are mentioned herein only by way of example and any further precursor compounds can be used which are suitable for the production of a VAM shell catalyst. It is particularly preferred that the precursor compounds are substantially chloride-free. By substantially chloride-free is meant that the empirical formula of the compound comprises no chloride, but it is not ruled out that the compound contains unavoidable chloride impurities for example due to production conditions.

It is particularly preferred that the Pd precursor compound in step (b) is the compound Pd(NH₃)₄(OH)₂, and the Au precursor compound is CsAuO₂.

To apply the acetate in step (a) of the method according to the invention, a solution containing the acetate is produced. Water is preferably used, more preferably deionized water, as solvent for producing the solution. The concentration of the acetate in the solution preferably lies in the range of from 0.5 to 5 mol/L, more preferably 1 to 3 mol/L, more preferably 1.5 to 2.5 mol/L and most preferably 1.9 to 2.1 mol/L.

The application of the solution containing the acetate can be carried out by any method known in the state of the art, such as for example wet-chemical impregnation, pore-filling method (incipient wetness) as well as by spray impregnation of any kind. Particularly preferably according to the invention, the acetate is applied to the support body by spray impregnation of the solution containing the acetate.

The support body can be present static, but is preferably moved. The movement of the support bodies can take place in any conceivable way, for example mechanically with a coating drum, mixing drum or also with the help of a support gas. It is particularly preferred that the movement of the support bodies during the spray impregnation is carried out with the help of a support gas (or process gas), for example in a fluid bed, a fluidized bed or in a static coating chamber, for example an Innojet AirCoater, wherein hot air is preferably blown in, with the result that the solvent is quickly evaporated. The temperature here is preferably 15 to 80°C, more preferably 20 to 60°C and most preferably 30 to 40°C. The advantage of this spray impregnation is that, depending on the temperature, an acetate-containing structure, which is homogeneously distributed over the volume to hollow sphere-shaped, forms in the volume of the support body with varying sphere thickness, wherein the surface layer of the support body which later bears noble metal can remain almost free of acetate and thus the noble metals can be taken up in the second application step. The spraying rate is preferably chosen during the spraying such that a balance is achieved between the evaporation rate of the solvent and the feed rate of the precursor compounds on the support body. It is particularly preferred that the spraying rate is constant during the spraying-in of the solution containing the acetate and lies in the range of the mass flow of from 5 to 25 g (solution)/min per 100 g of support body to be coated, more preferably in the range of from 10 to 20 g/min per 100 g and most preferably in the range of from 13 to 17 g/min per 100 g.

The solution containing acetate is preferably sprayed through a spray nozzle into the apparatus, in which the spray gas fed in, preferably air, is fed in at a pressure in the range of from 1 to 1.8 bar, more preferably 1.0 to 1.6 bar and most preferably 1.1 to 1.3 bar. Process air is preferably used as process gas for circulating the support bodies. The spraying rate and the pressure of the spraying air is preferably chosen for the nozzle used such that when it meets the circulating support bodies the droplet size of the resultant aerosol lies in the range of from 1 to 100 pm, more preferably in the range of from 10 to 40 pm. For example an IRN10 PEEK-type Rotojet spray nozzle from Innojet can be used.

The acetate anion loading preferably lies in the range of from 3 to 5.25 wt.-%, more preferably 3.3 to 4.5 wt.-% and most preferably 3.75 to 3.0 wt.-%, relative to the total weight of the support body dried after the application.

If the acetate is present as alkali acetate, the alkali metal loading lies in the corresponding stoichiometric ratio to the acetate anions.

After the application of the solution containing acetate, a drying is, according to the present invention, carried out in the temperature range of from 70 to 120°C, more preferably 80 to 110°C and most preferably 90 to 100°C in air, lean air or inert gas. The duration of the drying of the support bodies loaded with acetate preferably lies in the range of from 10 to 100 minutes, more preferably 30 to 60 minutes. The drying of the support body loaded with acetate can be carried out in a conventional drying device, but also in the coating device. If the drying is carried out in the coating device, drying preferably takes place such that the support bodies are present static therein, i.e. they are not moved. If a fluid bed or fluidized bed device is used, the support bodies are preferably not moved during the drying in the fluid bed or the fluidized bed.

In step (b) of the method according to the invention the Pd precursor compound and the Au precursor compound are preferably present dissolved in solution. The Pd precursor compound and the Au precursor compound can be present dissolved in a mixed solution, but they can also each be present in a separate solution. Pure solvents and solvent mixtures in which the selected metal compound(s) is/are soluble and which, after application to the catalyst support, can be easily removed again from same by means of drying are suitable as solvents for the transition metal precursor compounds. Preferred solvents are unsubstituted carboxylic acids, in particular acetic acid, ketones, such as acetone, and in particular deionized water. The application of the precursor compounds in step (b) of the method according to the invention to the support body can be carried out by methods known per se. It is conceivable that the precursor compound(s) is/are applied by steeping, the pore-filling method (incipient wetness) or by spray impregnation of the solution(s) containing the precursor compound(s). In step (b) the precursor compounds can be applied either from a mixed solution containing the Au precursor compound and the Pd precursor compound or from two solutions each containing one of the two precursor compounds. The precursor compounds are particularly preferably applied to the support body in step (b) simultaneously from two different solutions.

The application of the precursor compounds in step (b) of the method according to the invention is preferably carried out by spraying the support body with a solution containing the precursor compound. The support body is preferably moved in the same way as during the application of the acetate. Here too, it is preferred that the support bodies are moved in a process gas, e.g. in a coating drum, a fluid bed, a fluidized bed or in a static coating chamber of an Innojet AirCoater, wherein heated process gas is preferably blown in, with the result that the solvent is quickly evaporated. In this way, the precursor compounds are present in the named defined shell of the support body. The spraying rate is preferably chosen during the spraying such that a balance is achieved between the evaporation rate of the solvent and the feed rate of the precursor compounds on the support body. This makes it possible to set the desired shell thickness and palladium/gold distribution in the shell. Depending on the spraying rate, the shell thickness can thus be infinitely variably set and optimized, for example up to a thickness of 2 mm. But very thin shells with a thickness in the range of from 50 to 300 µm are thus also possible. If the Au precursor compound and the Pd precursor compound are applied from a mixed solution, the mixed solution is preferably conveyed from a receiver container via a pump to a spray nozzle via which the precursor compounds are sprayed onto the support bodies. If the Au precursor compound and the Pd precursor compound are applied from two separate solutions, it is preferred that these are stored in two separate receiver containers. They can then be conveyed from these by means of two pumps to two spray nozzles, with the result that the two solutions are sprayed in separately. Alternatively, the separate solutions can also be conveyed by means of two pumps to one spray nozzle, with the result that both solutions are sprayed in via one nozzle.

It is particularly preferred that the spraying rate in step (b) is constant and lies in the range of a mass flow (the solution containing the precursor compound) of from 5 g/min per 100 g to 25 g/min per 100 g of support body to be coated, more preferably in the range of from 10 to 20 g/min per 100 g and most preferably in the range of from 13 to 17 g/min per 100 g. In other words the ratio of the weight of the sprayed-on solution to the weight of the packed bed of the support body lies in the range of from 0.05 to 0.25, more preferably 0.1 to 0.2 and most preferably 0.13 to 0.17. A mass flow or ratio above the range indicated leads to catalysts with lower selectivity, a mass flow or ratio below the range indicated has no marked negative effects on the catalyst performance, but the catalyst production is very time-consuming and the production is thus inefficient.

If a fluid bed unit is used in step (a) and/or step (b) of the method according to the invention, it is preferred if the support bodies circulate elliptically or toroidally in the fluid bed. To give an idea of how the support bodies move in such fluid beds, it may be stated that in the case of "elliptical circulation" the support bodies move in the fluid bed in a vertical plane on an elliptical path, the size of the main and secondary axes changing. In the case of "toroidal" circulation the support bodies move in the fluid bed in a vertical plane on an elliptical path, the size of the main and secondary axes changing, and in a horizontal plane on a circular path, the size of the radius changing. On average, the support bodies move in a vertical plane on an elliptical path in the case of an "elliptical circulation", on a toroidal path in the case of a "toroidal circulation", i.e. a support body travels helically over the surface of the torus with a vertically elliptical section.

It is particularly preferred in the method according to the invention in step (b) that the application of the precursor compounds to the catalyst support body impregnated with acetate, thus e.g. with alkali acetate, is carried out by means of a fluid bed in a fluid bed unit. It is particularly preferred that the unit contains a so-called controlled air-glide layer. For one thing, the support bodies are thoroughly mixed by the controlled air-glide layer, wherein they simultaneously rotate about their own axis, and are dried evenly by the process air. For another, due to the consistent orbital movement, effected by the controlled air-glide layer, of the support bodies the support bodies pass through the spray procedure (application of the precursor compounds) at a virtually constant frequency (also applies to step (a) of the method according to the invention). A largely uniform shell thickness, or penetration depth of the metals into the support body, of a treated phase of support bodies is thereby achieved. A further result is that the noble metal concentration varies only relatively slightly over a relatively large area of the shell thickness, i.e. the noble metal concentration describes an approximately rectangular function over a large area of the shell thickness, whereby a largely uniform activity of the resulting catalyst is guaranteed over the thickness of the noble metal shell. Furthermore, the support body used in the method according to the invention is preferably heated during a spray impregnation in step (b), for example by means of heated process air. The process air here preferably has a temperature of from 10 to 110°C, more preferably 40 to 100°C and most preferably 50 to 90°C. The named upper limits should be adhered to in order to guarantee that the named outer shell has a small layer thickness with a high concentration of noble metal.

Air is preferably used as process air in the spray impregnation in steps (a) and/or (b). However, inert gases such as for example nitrogen, CO₂, helium, neon, argon or mixtures thereof can also be used.

If the Pd precursor compound and Au precursor compound in step (b) are applied from one solution, the solution preferably contains a proportion of Pd precursor compound such that Pd lies in the range of from 0.1 to 5 wt.-%, more preferably in the range of from 0.3 to 2 wt.-% and most preferably in the range of from 0.5 to 1 wt.-%, and a proportion of Au-containing precursor compound such that the proportion of Au lies in the range of from 0.05 to 10 wt.-%, more preferably in the range of from 0.1 to 5 wt.-% and most preferably in the range of from 0.1 to 1 wt.-%, in each case relative to the atomic weight proportion of the metals in solution.

If the Pd precursor compound and the Au precursor compound are applied separately from different solutions, the Pd-containing solution preferably contains Pd in the range of from 0.1 to 10 wt.-%, more preferably in the range of from 0.2 to 5 wt.-% and most preferably in the range of from 0.5 to 1 wt.-%, and the Au-containing solution preferably contains Au in the range of from 0.1 to 15 wt.-%, more preferably in the range of from 0.2 to 5 wt.-% and most preferably in the range of from 0.3 to 1 wt.-%, in each case relative to the atomic weight proportion of the metals in solution.

After the step of applying the precursor compounds to the support body in step (b), a drying step preferably takes place before the reducing step. The drying step is preferably carried out below the decomposition temperature of the precursor compounds, in particular at the temperatures mentioned further above, which are also mentioned for the drying of the applied acetate solution. The drying times as for the drying of the support body to which the acetate solution was applied also apply. By a decomposition temperature is meant the temperature at which the precursor compounds start to decompose. The drying preferably takes place either by process air in the fluid bed or fluidized bed device - if one is used - by standing in air or in a drying oven, preferably at a temperature in the range of from 60°C to 120°C. If the drying is carried out in the fluid bed apparatus or the fluidized bed apparatus, it is preferred that the support bodies are present static in the device, i.e. are not swirled by process air.

In addition to the step of simultaneously applying an Au precursor compound and a Pd precursor compound in step (b) of the method according to the invention, a pregilding can take place before step (b) and/or an aftergilding of the support body after step (b). The step of pregilding or the step of aftergilding is preferably carried out in the same way as in the step of applying the precursor compounds in step (b). Here, the same concentrations and the same precursor compounds as in the production of a solution produced separately in step (b) containing the Au precursor compound are preferably used. In principle, all possible application steps, namely steeping, pore-filling method or spray impregnation, as specified further above for step (b) are again also conceivable here. However, it is particularly preferred that the pregilding or aftergilding takes place by spray impregnation onto support bodies fluidized in a fluid bed or in a fluidized bed, as disclosed further above preferably also for step (b).

If a pregilding is carried out, an optional drying step, such as preferably takes place after step (b) as specified further above, can be carried out after this. A step of intermediate calcining can also be carried out after the pregilding, before step (b) is carried out. Likewise, in the case of the aftergilding after step (b), an identical drying or intermediate calcining step - as specified for the pregilding - can be carried out.

However, if a pre- and/or aftergilding is carried out, it is particularly preferred that this/these is/are carried out immediately before or immediately after the application of the precursor compounds in step (b). In this case, it is particularly preferred that the Pd precursor compound and the Au precursor compound are applied in separate solutions in step (b). Thus, for example, for a desired pregilding during the spray impregnation the Au precursor compound solution can be sprayed in to start with and the spraying-in of the Pd precursor compound solution can be initiated after a certain amount of time (while Au precursor compound solution continues to be sprayed on). In a similar way, the aftergilding can be carried out by spray impregnation such that in step (b) two separate solutions are sprayed in and the spraying-in of the Pd precursor compound solution ends before the spraying-in of the Au precursor compound solution is stopped. The pregilding has the advantage that during the simultaneous application of the two precursor compound solutions in step (b) these precursor compounds already attach to the applied Au precursor compound in the support body. In particular, the Au precursor compound solution is thereby prevented from penetrating further into the support body than the Pd precursor compound, with the result that both metals have an almost identical penetration depth in the finished catalyst. In the specified pregilding or aftergilding by spray impregnation, preferably the same specifications for the concentration of the Au precursor compound in the solution, the same spraying rate, the same spraying pressure, the same spraying air and the same process air apply as in step (b) of the method according to the invention.

If an aftergilding is carried out, the step of drying the support body is carried out, preferably not immediately after step (b), but preferably only after the step of applying the additional Au precursor compound in the step of aftergilding.

If the pregilding is carried out by spray impregnation in the preferably specified way before step (b), it is preferred that the spraying rate of the spraying-in of the Au precursor compound does not change at the start of the spraying-in of the Pd precursor compound solution. The ratio of the time interval of the simultaneous spraying-in of the two metal precursor compounds in step (b) to the time interval of the spraying-in of the Au precursor compound solution in the step of pre-impregnation preferably lies in the range of from 8 to 1, more preferably in the range of from 6 to 2 and most preferably in the range of from 5 to 3.

If an aftergilding is carried out by spray impregnation, the ratio of the time interval of the spraying-in of the two precursor compound solutions in step (b) to the time interval of the spraying-in of the Au precursor compound during the step of after-impregnation lies in the range of from 8 to 1, more preferably in the range of from 6 to 2 and most preferably in the range of from 5 to 3.
Here too, the spraying rate and the concentration of the Au precursor compound solution preferably do not change after the end of the spraying-in of the Pd precursor compound solution.

The spraying-on of the solutions containing the precursor compounds is effected in all the method steps of the method according to the invention preferably by atomizing the solution with the help of a spraying nozzle. Here, an annular gap nozzle is preferably used which sprays a spray cloud the plane of symmetry of which preferably runs parallel to the plane of the device base. Due to the 360° circumference of the spray cloud, the shaped bodies falling centrally can be sprayed particularly evenly with the solution. The annular gap nozzle, i.e. its orifice, is preferably completely embedded in the apparatus carrying out the circulating movement of the support bodies.

According to a further preferred embodiment of the method according to the invention, it is provided that the annular gap nozzle is centrally arranged in the base of the apparatus carrying out the circulating movement of the support bodies and the orifice of the annular gap nozzle is completely embedded in the apparatus. It is thereby guaranteed that the free path of the drops of the spray cloud until they meet a shaped body is relatively short and, accordingly, relatively little time remains for the drops to coalesce into larger drops, which could work against the formation of a largely uniform shell thickness.

After the optional step of drying after the step/steps of applying the precursor compounds, the reduction of the metal components of the precursor compounds to the elemental metals takes place in step (c). The step of reduction is preferably carried out by a temperature treatment in a non-oxidizing atmosphere for the reduction of the metal components of the precursor compounds to the elemental metals. The temperature treatment in a non-oxidizing atmosphere is preferably carried out in a temperature range of from 50 to 400°C, more preferably 50 to 200°C and most preferably 60 to 150°C.

It is particularly preferred that the temperature treatment is carried out in a non-oxidizing atmosphere likewise in the coating device directly after the drying step after the coating with the metal precursor compounds, in particular without the support bodies being removed from the coating device between these steps. This leads to a significant time advantage as well as less abrasion because the time and mechanical strain associated with the decanting of the support bodies cease to apply.

By a non-oxidizing atmosphere is meant in the present invention an atmosphere which contains no, or almost no, oxygen or other gases with an oxidizing action. The non-oxidizing atmosphere can be an atmosphere of inert gas or a reducing atmosphere. A reducing atmosphere can be formed by a gas with a reductive action or a mixture of gas with a reductive action and inert gas.

In a variant of the method according to the invention, the reduction is carried out in an inert gas atmosphere. In this case, the counterions of the metal in the metal-containing precursor compound have a reductive action. A person skilled in the art is sufficiently aware which counterions can have a reductive action in this case.

In a further variant of the method according to the invention, the temperature treatment can be carried out directly in a reducing atmosphere. In this case, the precursor compounds are decomposed at the same temperature of the temperature treatment and the metal component is reduced to the elemental metals.

In yet another variant of the method according to the invention, the temperature treatment is preferably carried out such that there is a change from an inert gas atmosphere to a reducing atmosphere during the temperature treatment. The precursor compounds are first decomposed at their decomposition temperature in an inert gas atmosphere and then the metal components are reduced to the elemental metals by the change to a reducing atmosphere. The temperature during the decomposition under inert gas preferably lies in the range of from 200 to 400°C. The temperature during the subsequent reduction then preferably lies in the above-specified range.

According to the invention, it is particularly preferred that the change from an inert gas atmosphere to a reducing atmosphere is carried out such that the temperature during the change does not fall below the temperature desired for the reduction.

N₂, He, Ne, Ar or mixtures thereof for example are used as inert gas. N₂ is particularly preferably used.

The component with a reductive action in the reducing atmosphere is normally to be selected depending on the nature of the metal component to be reduced, but preferably selected from the group of gases or vaporizable liquids consisting of ethylene, hydrogen, CO, NH₃, formaldehyde, methanol, formic acid and hydrocarbons, or is a mixture of two or more of the above-named gases/liquids. The reducing atmosphere particularly preferably comprises hydrogen as reducing component. It is preferred in particular if the reducing atmosphere is formed from forming gas, a mixture of N₂ and H₂. The hydrogen content is in the range of from 1 vol.-% to 15 vol.-%. The method according to the invention is [reduced] for example with hydrogen (4 to 5 vol.-%) in nitrogen as process gas at a temperature in the range of from 60 to 150°C over a period of for example from 0.25 to 10 hours, preferably 2 to 6 hours.

The change named in the second method alternative from inert gas to a reducing atmosphere during the step of reduction preferably takes place by feeding one of the named reducing components into an inert gas atmosphere. Hydrogen gas is preferably fed in. The feeding of a gas with a reductive action to the inert gas has the advantage that the temperature does not fall substantially, or not down to or below the lower limit of 60°C desired for the reduction, with the result that there is no need for another cost- and energy-intensive heating necessitated by a corresponding total atmosphere exchange.

The catalyst support obtained after step (c) preferably contains a proportion of Pd in the range of from 0.5 to 2.5 wt.-%, more preferably in the range of from 0.7 to 2 wt.-%, even more preferably in the range of from 0.9 to 1.5 wt.-%, relative to the total weight of the catalyst support body after the reduction.

The catalyst support body obtained after step (c) preferably contains a proportion of Au in the range of from 0.1 to 1.0 wt.-%, more preferably in the range of from 0.2 to 0.8 wt.-% and most preferably in the range of from 0.3 to 0.6 wt.-%, relative to the total weight of the catalyst support body after the reduction.

It is particularly preferred in the method according to the invention that steps (a) and (b) and the optional steps of pre- or aftergilding and the optional steps of drying are carried out in a single device. Furthermore, it can also be preferred that the step (c) of reduction is also carried out in the same device as steps (a) and (b). This has the advantage over other methods that the noble metal yield was able to be greatly optimized, as the abrasion by the introduction and removal of the support bodies into and from different apparatuses between the steps was no longer necessary. Thus, for example with a method according to the invention in which all the steps were carried out in one fluid bed apparatus the loss of noble metal was only 3 wt.-%.

It is particularly preferred that the device in which steps (a) and (b) and optionally also step (c) are carried out is constituted by suitable conventional coating drums, fluidized bed devices or fluid bed devices. Suitable conventional coating drums, fluidized bed devices or fluid bed devices for carrying out the application of the precursor compound in the method according to the invention are known in the state of the art and are marketed for example by companies such as Heinrich Brucks GmbH (Alfeld, Germany), ERWEKA GmbH (Heusenstamm, Germany), Stechel (Germany), DRIAM Anlagenbau GmbH (Eriskirch, Germany), Glatt GmbH (Binzen, Germany), G. S. Divisione Verniciatura (Osteria, Italy), HOFER-Pharma Maschinen GmbH (Weil am Rhein, Germany), L.B. Bohle Maschinen und Verfahren GmbH (Enningerloh, Germany), Lödige Maschinenbau GmbH (Paderborn, Germany), Manesty (Merseyside, Great Britain), Vector Corporation (Marion (IA) USA), Aeromatic-Fielder AG (Bubendorf, Switzerland), GEA Process Engineering (Hampshire, Great Britain), Fluid Air Inc. (Aurora, Illinois, USA), Heinen Systems GmbH (Varel, Germany), Huttlin GmbH (Steinen, Germany), Umang Pharmatech Pvt. Ltd. (Maharashtra, India) and Innojet Technologies (Lörrach, Germany). Particularly preferred fluid bed equipment is sold with the name Innojet® AirCoater or Innojet® Ventilus by Innojet Technologies. Here the IAC-5 coater, the IAC-150 coater or the IAC-025 coater, all from the company Innojet, is particularly preferably used.

A further subject of the present invention is also a shell catalyst which can be obtained using the method according to the invention. The shell catalyst according to the invention differs from conventional shell catalysts for the synthesis of VAM in that it has a significantly higher selectivity and activity in the synthesis of VAM. This is to be attributed to the application of the acetate before the application of the metal precursor compounds and before the reduction. The structural differences clearly present in respect of the better selectivity and activity of the shell catalyst according to the invention compared with conventional catalysts cannot be expressed in physical values at the time of the application. The shell catalyst according to the invention can therefore only be distinguished from conventional catalysts by the manner of its production and the established increased selectivity and activity.

Another embodiment relates to the use of a shell catalyst produced using the method according to the invention for producing alkenyl carboxylic acid esters, in particular VAM and/or allyl acetate monomer. In other words the present invention also relates to a method for producing VAM or allyl acetate in which acetic acid, ethylene or propylene and oxygen or oxygen-containing gases are passed over the catalyst according to the invention. Generally this takes place by passing acetic acid, ethylene and oxygen or oxygen-containing gases over the catalyst according to the invention at temperatures of from 100 to 200°C, preferably 120 to 200°C, and at pressures of from 1 to 25 bar, preferably 1 to 20 bar, wherein non-reacted educts can be recycled. Expediently, the oxygen concentration is kept below 10 vol.-%. Under certain circumstances, however, a dilution with inert gases such as nitrogen or carbon dioxide is also advantageous. Carbon dioxide is particularly suitable for dilution as it is formed in small quantities in the course of VAM synthesis and collects in the recycle gas. The formed vinyl acetate is isolated with the help of suitable methods, which are described for example in US 5,066,365 A. Equivalent methods have also been published for allyl acetate.

The invention is described in more detail below using a figure and embodiment examples without these being understood as limiting.

Figure:
- Figure 1:: Figure 1 shows the quantity of VAM molecules produced per molecule of oxygen as a function of the CO₂/O₂ ratio when a catalyst produced according to Example 1 and comparison example 1 is used in the catalytic synthesis of VAM.
- Figure 2:: Figure 2 shows the space-time yield (STY) of VAM as a function of the O₂ conversion when a catalyst produced according to Example 1 and comparison example 1 is used in the catalytic synthesis of VAM.
- Figure 3:: Figure 3 shows the quantity of VAM molecules produced per molecule of oxygen as a function of the CO₂/O₂ ratio when a catalyst produced according to Example 2 and comparison example 2 is used in the catalytic synthesis of VAM.
- Figure 4:: Figure 4 shows the space-time yield (STY) of VAM as a function of the O₂ conversion when a catalyst produced according to Example 2 and comparison example 2 is used in the catalytic synthesis of VAM.

### Examples:

### Example 1: Production of a catalyst A:

Firstly, 100 g of the support "KA-Zr-14" (with 14% ZrO₂-doped KA-160 support from Süd-Chemie) was calcined for 4 h at 750°C. Then, the support bodies were presented in a fluid bed in an AirCoater 025-type coater from Innojet using air as process gas for 10 min to remove dust. [Onto the fluidized support bodies] 20.32 g of a 2 molar KOAc solution was then sprayed onto the swirled support bodies at a spraying rate of 15 g/min/100 g support bodies at 33°C. Then, the support bodies were presented static and dried at 88°C for 35 min. Renewed fluidizing of the support bodies after the drying took place again with the help of air as process gas. Firstly, an aqueous solution containing KAuO₂ (produced by mixing 4.05g of a 3.6% Au solution + 50 ml water) was sprayed onto the swirled support bodies at 70°C at a spraying rate of 15 g solution/min/100 g support bodies. Directly afterwards, two aqueous solutions were sprayed in parallel, wherein one solution contains KAuO₂ (produced by mixing 4.05 g of a 3.6% Au solution with 50 ml water) and the other solution contains Pd(NH₃)₄(OH)₂ (produced by mixing 32.58 g of a 4.7% Pd solution + 50 ml water). Both solutions were sprayed in at a spraying rate of approximately 15 g solution/min/100 g support bodies at a temperature of 70°C. Directly afterwards, an aqueous solution containing KAuO₂ (produced by mixing 4.05 g of a 3.6% Au solution + 50 ml water) was sprayed on at 70°C at a spraying rate of 15 g solution/min/100 g support bodies. Then, the support bodies were presented static in the coater again and dried in this state at 90°C for 35 min.

The support bodies were then reduced for 25 min at 70°C with forming gas (98% N₂ and 2% H₂).

According to ICP-AES analysis, the proportion of Pd was 1.4 wt.-% and the proportion of Au was 0.4 wt.-%, in each case relative to the total mass of the support.

### Comparison example 1: Production of a catalyst B:

Firstly, 100 g of the support "KA-Zr-14" (with 14% ZrO₂-doped KA-160 support from Süd-Chemie) was calcined for 4 h at 750°C. Then, the support bodies were transferred to an AirCoater 025-type coater and presented fluidized with the help of air as process gas. Firstly, an aqueous solution containing KAuO₂ (produced by mixing 4.05 g of a 3.6% Au solution + 50 ml water) was sprayed onto the swirled support bodies at 70°C at a spraying rate of 15 g solution/min/100 g support bodies. Directly afterwards, two aqueous solutions were sprayed in parallel, wherein one solution contains KAuO₂ (produced by mixing 4.05 g of a 3.6% Au solution with 50 ml water) and the other solution contains Pd(NH₃)₄(OH)₂ (produced by mixing 32.58 g of a 4.7% Pd solution + 50 ml water). Both solutions were sprayed in at a spraying rate of approximately 15 g solution/min/100 g support bodies at a temperature of 70°C. Directly afterwards, an aqueous solution containing KAuO₂ (produced by mixing 4.05 g of a 3.6% Au solution + 50 ml water) was sprayed on at 70°C at a spraying rate of 15 g solution/min/100 g support bodies. Then, the support bodies were presented static in the coater again and dried in this state at 90°C for 35 min.

Then, the support bodies were removed from the coater and transferred to a reduction furnace in which they were reduced for 4 h at 150°C with H₂.

Then, the support bodies were impregnated with KOAc by means of the incipient wetness method, by allowing a 2-molar KOAc solution to work on them.

According to ICP-AES analysis, the proportion of Pd was 1.4 wt.-% and the proportion of Au was 0.4 wt.-%, in each case relative to the total mass of the support.

### Example 2: Production of a catalyst C:

100 g of a KA-160 support (obtainable from Sud-Chemie AG) was impregnated with a KOAc solution (20.58 g 2 molar KOAc solution and 42.13 g distilled water) according to the incipient wetness method. Then, the support bodies were dried in the fluidized bed drier for 45 min at 90°C. 31.38 g Pd(NH₃)₄(OH)₂ solution (4.7%) and 10.89 g KAuO₂ solution (3.6%) diluted with 50 ml dist. water were applied as mixed solution to a KA-160 support (obtainable from Süd-Chemie AG) at a spraying rate of 15 g solution per minute per 100 g support bodies at a temperature of 70°C. The support bodies were swirled in an AirCoater 025-type Innojet Air-Coater by means of air as process gas. The obtained support was dried at 90°C for 45 minutes in the static state in the coater. Then, the sample was reduced at 150°C for 4 hours in the gas phase with forming gas (3 vol.-% H₂ in N₂).

According to ICP-AES analysis, the proportion of Pd was 1.4 wt.-% and the proportion of Au was 0.4 wt.-%, in each case relative to the total mass of the support.

### Comparison example 2: Production of a catalyst D:

31.38 g Pd(NH₃)₄(OH)₂ solution (4.7%) and 10.89 g KAuO₂ solution (3.6%) diluted with 50 ml dist. water were applied as mixed solution to a KA-160 support (obtainable from Süd-Chemie AG) at a spraying rate of 15 g solution per minute per 100 g support bodies at a temperature of 70°C. The support bodies were swirled in an AirCoater 025-type Innojet Air-Coater by means of air as process gas. The obtained support was dried at 90°C for 45 minutes in the static state in the coater. Then, the sample was reduced at 150°C for 4 hours in the gas phase with forming gas (3 vol.-% H₂ in N₂). After the reduction, the catalyst was impregnated with a KOAc solution (1.95 g 2 molar KOAc solution and 3.99 g distilled water) according to the incipient wetness method. The sample was then dried at 90°C for 45 minutes in the static state in the fluidized bed drier.

According to ICP-AES analysis, the proportion of Pd was 1.4 wt.-% and the proportion of Au was 0.4 wt.-%, in each case relative to the total mass of the support.

### Example 2: Test results for catalysts A to D in respect of their activity and selectivity in the synthesis of VAM:

For this, acetic acid, ethylene and oxygen were each passed over the catalysts A and B at a temperature of 140°C/12 hours → 143°C/12 hours → 146°C/12 hours (these are the respective reaction temperatures that apply according to the sequence during the automated execution of the screening protocol, i.e. measurement is carried out for 12 hours at 140°C, then for 12 hours at 143°C, and then for 12 hours at 146°C reactor temperature) and a pressure of 6.5 bar. The concentrations of the components used were: 39% ethylene, 6% O₂, 0.6% CO₂, 9% methane, 12.5% acetic acid, remainder N₂.

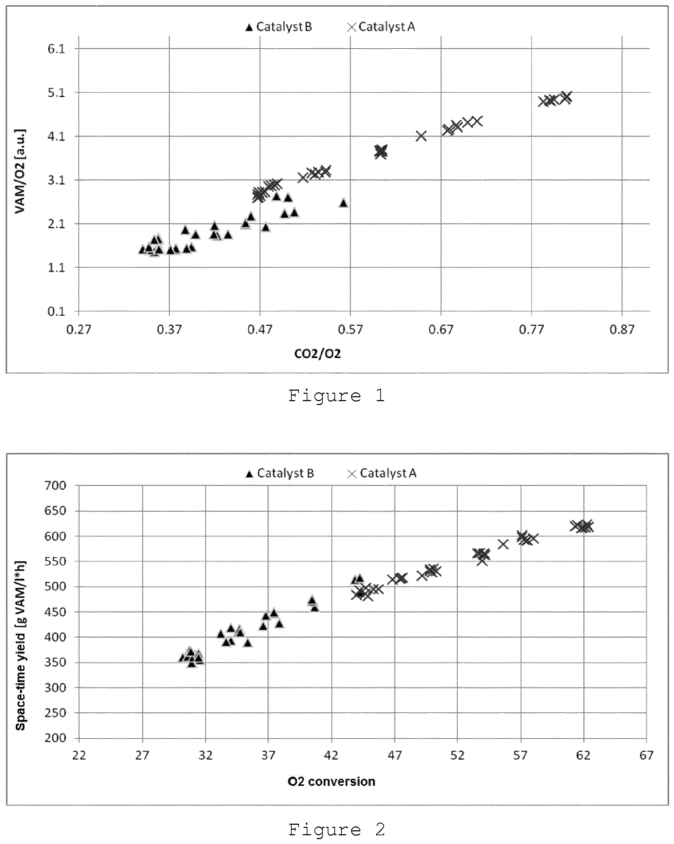

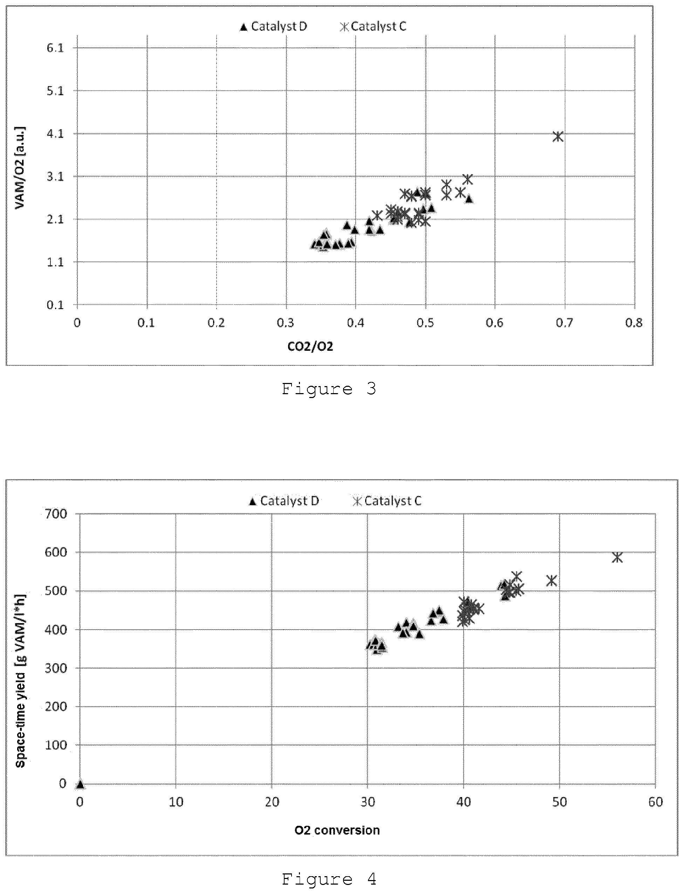

Figures 1 and 2 show the selectivity or the activity of catalysts A and B as a function of the O₂ conversion. Figures 3 and 4 show the selectivity or the activity of catalysts C and D as a function of the O₂ conversion. The values are also listed in tabular form in the following Tables 1 to 4:

**Table 1:**

| Catalyst A | | | |
|---|---|---|---|
| CO₂/O₂ | VAM/O₂ | O₂ conversion | Space-time yield (g VAM/1h) |
| 0.5 | 2.7 | 44.9 | 480.7 |
| 0.5 | 2.7 | 44.7 | 486.7 |
| 0.5 | 2.7 | 44.0 | 483.1 |
| 0.5 | 2.8 | 44.2 | 492.9 |
| 0.5 | 2.8 | 45.3 | 494.8 |
| 0.5 | 2.8 | 45.7 | 494.6 |
| 0.5 | 2.8 | 44.7 | 497.9 |
| 0.5 | 3.3 | 49.8 | 530.1 |
| 0.5 | 3.3 | 50.1 | 535.7 |
| 0.5 | 3.3 | 50.3 | 531.0 |
| 0.5 | 3.3 | 49.8 | 534.0 |
| 0.5 | 3.2 | 50.0 | 527.7 |
| 0.5 | 3.1 | 49.2 | 521.8 |
| 0.6 | 3.8 | 53.7 | 566.9 |
| 0.6 | 3.8 | 54.2 | 562.7 |
| 0.6 | 3.8 | 54.1 | 565.9 |
| 0.6 | 3.8 | 54.0 | 563.7 |
| 0.6 | 3.7 | 53.9 | 550.4 |
| 0.6 | 3.8 | 53.6 | 566.7 |
| 0.6 | 4.1 | 55.6 | 583.9 |
| 0.7 | 4.5 | 58.0 | 595.1 |
| 0.7 | 4.4 | 57.1 | 601.4 |
| 0.7 | 4.4 | 57.1 | 598.5 |
| 0.7 | 4.3 | 57.5 | 592.3 |
| 0.7 | 4.3 | 57.5 | 591.5 |
| 0.7 | 4.2 | 57.3 | 593.2 |
| 0.8 | 5.0 | 62.3 | 623.1 |
| 0.8 | 5.0 | 62.1 | 617.4 |
| 0.8 | 5.0 | 62.4 | 617.8 |
| 0.8 | 4.9 | 61.9 | 617.1 |
| 0.8 | 4.9 | 61.8 | 615.9 |

**Table 2:**

| Catalyst B | | | |
|---|---|---|---|
| CO/O₂ | VAM/O₂ | O₂ conversion | Space-time yield (g VAM/1h) |
| 0.37 | 1.91 | 37.68 | 410.4 |
| 0.37 | 1.94 | 38.9 | 413.84 |
| 0.38 | 1.96 | 39.28 | 414.82 |
| 0.42 | 2.26 | 42.89 | 449.37 |
| 0.42 | 2.27 | 43.41 | 448.08 |
| 0.42 | 2.28 | 43.15 | 451.97 |
| 0.42 | 2.29 | 43.42 | 451.07 |
| 0.43 | 2.35 | 43.68 | 459.91 |
| 0.43 | 2.35 | 44.28 | 455.77 |
| 0.47 | 2.58 | 46.83 | 477.55 |
| 0.47 | 2.61 | 47.09 | 480.28 |
| 0.47 | 2.61 | 46.56 | 484.24 |
| 0.47 | 2.62 | 47.22 | 480.06 |
| 0.48 | 2.62 | 47.36 | 479.17 |
| 0.48 | 2.62 | 46.94 | 481.09 |
| 0.48 | 2.62 | 46.82 | 480.75 |
| 0.53 | 2.93 | 49.97 | 505.31 |
| 0.54 | 2.95 | 50.27 | 504.67 |
| 0.54 | 2.95 | | 502.98 |
| 0.52 | 2.99 | | 498.56 |
| 0.52 | 2.88 | | 498.85 |
| 0.52 | 2.87 | 49.55 | 498.99 |
| 0.52 | 2.88 | 49.89 | 497.08 |
| 0.59 | 3.21 | 53.17 | 517.84 |
| 0.59 | 3.22 | 53.45 | 515.59 |
| 0.59 | 3.19 | 53.03 | 516.06 |
| 0.58 | 3.19 | 52.94 | 515.34 |
| 0.59 | 3.22 | 53.04 | 519.72 |
| 0.58 | 3.17 | 52.73 | 513.56 |
| 0.58 | 3.14 | 52.55 | 511.64 |
| 0.41 | 2.2 | 41.95 | 439.28 |

**Table 3:**

| Catalyst C | | | |
|---|---|---|---|
| CO/O₂ | VAM/O₂ | O₂ conversion | Space-time yield (g VAM/1h) |
| 0.48 | 2.02 | 39.9 | 421.03 |
| 0.5 | 2.04 | 40.08 | 424.82 |
| 0.49 | 2.08 | 40.56 | 428.84 |
| 0.46 | 2.1 | 39.84 | 435.44 |
| 0.46 | 2.16 | 39.98 | 447.28 |
| 0.47 | 2.22 | 40.31 | 456.89 |
| 0.49 | 2.21 | 40.54 | 452.89 |
| 0.43 | 2.18 | 40.22 | 449.46 |
| 0.46 | 2.28 | 40.79 | 464.38 |
| 0.47 | 2.25 | 41 | 456.54 |
| 0.45 | 2.23 | 40.99 | 452.31 |
| 0.49 | 2.25 | 41.57 | 453.69 |
| 0.45 | 2.32 | 40 | 472.01 |
| 0.53 | 2.67 | 45.41 | 501.13 |
| 0.5 | 2.73 | 44.8 | 515.54 |
| 0.48 | 2.63 | 44.63 | 497.54 |
| 0.55 | 2.73 | 45.76 | 506.05 |
| 0.56 | 3.03 | 49.14 | 527.16 |
| 0.69 | 4.04 | 56 | 587 |
| 0.47 | 2.69 | 44.42 | 504.15 |
| 0.5 | 2.66 | 44.77 | 496.89 |
| 0.53 | 2.91 | 45.49 | 537.29 |
| 0.48 | 2.64 | 44.73 | 496.38 |

**Table 4:**

| Catalyst D | | | |
|---|---|---|---|
| CO/O₂ | VAM/O₂ | O₂ conversion | Space-time yield (g VAM/lh) |
| 0.31 | 1.21 | 27.35 | 303.85 |
| 0.35 | 1.25 | 28.47 | 309.68 |
| 0.37 | 1.38 | 29.04 | 339.25 |
| 0.34 | 1. 44 | 28.80 | 354.44 |
| 0.34 | 1.35 | 28.85 | 331.39 |
| 0.36 | 1.44 | 29.89 | 347.01 |
| 0.34 | 1. 42 | 29.84 | 343.49 |
| 0.39 | 1.43 | 30.57 | 342.34 |
| 0.34 | 1. 46 | 30.56 | 349.29 |
| 0.36 | 1.48 | 30.75 | 352.59 |
| 0.38 | 1.51 | 31.61 | 354.71 |
| 0.36 | 1.53 | 31.58 | 360.97 |
| 0.41 | 1.83 | 34.85 | 411.48 |
| 0.38 | 1.71 | 34.04 | 386.60 |
| 0.43 | 1.86 | 35.19 | 412.62 |
| 0.42 | 2.03 | 37.70 | 433.66 |
| 0.44 | 2.01 | 38.35 | 424.94 |
| 0.51 | 2.36 | 41.48 | 468.41 |
| 0.46 | 2.36 | 41.41 | 469.33 |
| 0.52 | 2.28 | 41.80 | 450.27 |
| 0.49 | 2.39 | 41.55 | 474.80 |
| 0.51 | 2.64 | 45.47 | 486.80 |
| 0.38 | 1.94 | 34.97 | 424.49 |
| 0.39 | 1.87 | 34.89 | 412.60 |
| 0.39 | 1.81 | 35.26 | 398.06 |

As can be seen from the comparison of the values from Tables 1 and 2 and Figures 1 and 2, the catalyst A produced according to the invention has a substantially higher selectivity and activity (O₂ conversion rate) than the comparison catalyst B. Likewise, the catalyst C according to the invention has a substantially higher selectivity and activity (O₂ conversion rate) than the comparison catalyst D (see Tables 3 and 4 and Figures 3 and 4).

## Claims

1. Method for producing a shell catalyst, comprising the following steps:
(a) applying a solution containing an acetate to a support body, wherein the acetate contains an acetate anion, and afterwards drying the support body at a temperature in the range of from 70 to 120 °C;
(b) applying a Pd precursor compound and an Au precursor compound to a support body obtained after step (a) and impregnated with the acetate; and
(c) reducing the metal components of the precursor compounds of the support body obtained after step (b) to the elemental metals.

2. Method according to claim 1, wherein the acetate is an alkali acetate.

3. Method according to claim 1 or 2, wherein the Pd precursor compound is a hydroxo complex.

4. Method according to one of claims 1 to 3, wherein the Au precursor compound is an aurate.

5. Method according to one of claims 1 to 4, wherein the application of the acetate takes place by spray impregnation of a solution containing the acetate.

6. Method according to one of claims 1 to 5, wherein the application of the Pd precursor compound and of the Au precursor compound takes place simultaneously by spray impregnation of a mixed solution containing both precursor compounds or by spray impregnation of two solutions each containing one of the precursor compounds.

7. Method according to one of claims 1 to 6, wherein before step (b) an Au precursor compound is additionally applied to the support body obtained after step (a).

8. Method according to one of claims 1 to 7, wherein before step (c) an Au precursor compound is additionally applied to the support body obtained after step (b).

9. Method according to claim 7 or 8, wherein the application of the Au precursor compound takes place by spray impregnation of a solution containing the precursor compound.

10. Method according to claim 5, 6 or 9, wherein the support body is moved in a fluid bed or in a fluidized bed during the spray impregnation.

11. Method according to one of claims 1 to 10, wherein the reduction of the metal components is carried out in a non-oxidizing atmosphere.

12. Method according to claim 11, wherein the non-oxidizing atmosphere contains hydrogen or ethylene as reducing agent.

13. Method according to one of claims 1 to 12, wherein steps (a) and (b), or (a), (b) and (c) are carried out in a single device.

14. Shell catalyst obtainable using a method according to one of claims 1 to 12.

15. Use of a shell catalyst according to claim 14 or of a shell catalyst produced according to one of claims 1 to 13 for producing alkenyl carboxylic acid esters.

## Patentansprüche

1. Verfahren zur Herstellung eines Schalenkatalysators, umfassend die folgenden Schritte:
(a) Aufbringen einer ein Acetat enthaltenden Lösung auf einen Trägerkörper, wobei das Acetat ein Acetatanion enthält, und anschließendes Trocknen des Trägerkörpers bei einer Temperatur im Bereich von 70 bis 120 °C,
(b) Aufbringen einer Pd-Vorläuferverbindung und einer Au-Vorläuferverbindung auf einen nach Schritt (a) erhaltenen und mit dem Acetat imprägnierten Trägerkörper und
(c) Reduzieren der Metallkomponenten der Vorläuferverbindungen des nach Schritt (b) erhaltenen Trägerkörpers zu den elementaren Metallen.

2. Verfahren nach Anspruch 1, wobei es sich bei dem Acetat um ein Alkaliacetat handelt.

3. Verfahren nach Anspruch 1 oder 2, wobei es sich bei der Pd-Vorläuferverbindung um einen Hydroxokomplex handelt.

4. Verfahren nach einem der Ansprüche 1 bis 3, wobei es sich bei der Au-Vorläuferverbindung um ein Aurat handelt.

5. Verfahren nach einem der Ansprüche 1 bis 4, wobei das Aufbringen des Acetats mittels Sprühimprägnierung einer das Acetat enthaltenden Lösung erfolgt.

6. Verfahren nach einem der Ansprüche 1 bis 5, wobei das Aufbringen der Pd-Vorläuferverbindung und der Au-Vorläuferverbindung gleichzeitig mittels Sprühimprägnierung einer gemischten Lösung, die beide Vorläuferverbindungen enthält, oder mittels Sprühimprägnierung von zwei Lösungen, die jeweils eine der Vorläuferverbindungen enthalten, erfolgt.

7. Verfahren nach einem der Ansprüche 1 bis 6, wobei vor Schritt (b) zusätzlich eine Au-Vorläuferverbindung auf den nach Schritt (a) erhaltenen Trägerkörper aufgebracht wird.

8. Verfahren nach einem der Ansprüche 1 bis 7, wobei vor Schritt (c) zusätzlich eine Au-Vorläuferverbindung auf den nach Schritt (b) erhaltenen Trägerkörper aufgebracht wird.

9. Verfahren nach Anspruch 7 oder 8, wobei das Aufbringen der Au-Vorläuferverbindung mittels Sprühimprägnierung einer die Vorläuferverbindung enthaltenden Lösung erfolgt.

10. Verfahren nach Anspruch 5, 6 oder 9, wobei der Trägerkörper während der Sprühimprägnierung in einem Fließbett oder in einer Wirbelschicht bewegt wird.

11. Verfahren nach einem der Ansprüche 1 bis 10, wobei die Reduktion der Metallkomponenten in einer nichtoxidierenden Atmosphäre durchgeführt wird.

12. Verfahren nach Anspruch 11, wobei die nichtoxidierende Atmosphäre Wasserstoff oder Ethylen als Reduktionsmittel enthält.

13. Verfahren nach einem der Ansprüche 1 bis 12, wobei die Schritte (a) und (b) oder (a), (b) und (c) in einer einzigen Vorrichtung durchgeführt werden.

14. Schalenkatalysator, erhältlich unter Verwendung eines Verfahrens nach einem der Ansprüche 1 bis 12.

15. Verwendung eines Schalenkatalysators nach Anspruch 14 oder eines nach einem der Ansprüche 1 bis 13 hergestellten Schalenkatalysators zur Herstellung von Alkenylcarbonsäureestern.

## Revendications

1. Procédé de production d'un catalyseur à enveloppe, comprenant les étapes suivantes :
(a) application d'une solution contenant un acétate sur un corps de support, dans lequel l'acétate contient un anion acétate, puis séchage du corps de support à une température dans la plage de 70 à 120 °C ;
(b) application d'un composé précurseur de Pd et d'un composé précurseur de Au sur un corps de support obtenu après l'étape (a) et imprégné avec l'acétate ; et
(c) réduction des composants métalliques des composés précurseurs du corps de support obtenu après l'étape (b) en métaux élémentaires.

2. Procédé selon la revendication 1, dans lequel l'acétate est un acétate d'alcali.

3. Procédé selon la revendication 1 ou 2, dans lequel le composé précurseur de Pd est un complexe hydroxo.

4. Procédé selon l'une des revendications 1 à 3, dans lequel le composé précurseur de Au est un aurate.

5. Procédé selon l'une des revendications 1 à 4, dans lequel l'application de l'acétate est conduite par imprégnation par pulvérisation d'une solution contenant l'acétate.

6. Procédé selon l'une des revendications 1 à 5, dans lequel l'application du composé précurseur de Pd et du composé précurseur de Au est conduite simultanément par imprégnation par pulvérisation d'une solution mixte contenant les deux composés précurseurs ou par imprégnation par pulvérisation de deux solutions contenant chacune un des composés précurseurs.

7. Procédé selon l'une des revendications 1 à 6, dans lequel, avant l'étape (b), un composé précurseur de Au est en outre appliqué sur le corps de support obtenu après l'étape (a).

8. Procédé selon l'une des revendications 1 à 7, dans lequel, avant l'étape (c), un composé précurseur de Au est en outre appliqué sur le corps de support obtenu après l'étape (b).

9. Procédé selon la revendication 7 ou 8, dans lequel l'application du composé précurseur de Au est conduite par imprégnation par pulvérisation d'une solution contenant le composé précurseur.

10. Procédé selon la revendication 5, 6 ou 9, dans lequel le corps de support est déplacé dans un lit fluide ou dans un lit fluidisé pendant l'imprégnation par pulvérisation.

11. Procédé selon l'une des revendications 1 à 10, dans lequel la réduction des composants métalliques est conduite dans une atmosphère non oxydante.

12. Procédé selon la revendication 11, dans lequel l'atmosphère non oxydante contient de l'hydrogène ou de l'éthylène en tant qu'agent réducteur.

13. Procédé selon l'une des revendications 1 à 12, dans lequel les étapes (a) et (b), ou (a), (b) et (c) sont conduites dans un dispositif unique.

14. Catalyseur à enveloppe pouvant être obtenu au moyen d'un procédé selon l'une des revendications 1 à 12.

15. Utilisation d'un catalyseur à enveloppe selon la revendication 14 ou d'un catalyseur à enveloppe produit selon l'une des revendications 1 à 13 pour produire des esters d'acide alcénylcarboxylique.
